# EUROPÄISCHE PATENTSCHRIFT

(11) **EP 1 246 624 B1**
(45) Veröffentlichungstag und Bekanntmachung des Hinweises auf die Patenterteilung: **29.06.2005**
(21) Anmeldenummer: 00977586.7
(22) Anmeldetag: 21.11.2000
(51) Int. Cl.: A61K 31/53, A01N 43/707, A01N 43/66, A61P 33/02

(54) **TRIAZINONVERBINDUNGEN ZUR BEHANDLUNG VON DURCH DEN BEFALL MIT PARASITISCHEN PROTOZOEN BEDINGTEN KRANKHEITEN**
TRIAZINONE COMPOUNDS FOR TREATING DISEASES RESULTING FROM INFESTATION WITH PARASITIC PROTOZOANS
COMPOSES DE TRIAZINONE DESTINES AU TRAITEMENT DE MALADIES DUES A L'INFECTION PAR DES PROTOZOAIRES PARASITAIRES

(30) Priorität: 03.12.1999 DE 19958388
(43) Veröffentlichungstag der Anmeldung: 09.10.2002
(73) Patentinhaber: Bayer HealthCare AG, 51368 Leverkusen (DE)
(72) Erfinder: GREIF, Gisela, 53424 Remagen (DE)
(86) Internationale Anmeldenummer: PCT/EP2000/011837
(87) Internationale Veröffentlichungsnummer: WO 2001/039778

(56) Entgegenhaltungen:
- WO-A-00/19964
- WO-A-00/37063
- WO-A-00/37064
- US-A- 5 830 893
- US-A- 5 883 095
- DATABASE BIOSIS [Online] BIOSCIENCES INFORMATION SERVICE, PHILADELPHIA, PA, US; 1996 HABERKORN A: "Chemotherapy of human and animal coccidioses: State and perspectives." Database accession no. PREV199698772552 XP002168492 & PARASITOLOGY RESEARCH, Bd. 82, Nr. 3, 1996, Seiten 193-199, ISSN: 0932-0113
- DATABASE CHEMABS [Online] CHEMICAL ABSTRACTS SERVICE, COLUMBUS, OHIO, US; AOKI, TOYOHARU: "Effect of sulfamethopyrazine and combined use of vaccine on acute toxoplasmosis in mice" retrieved from STN Database accession no. 76:10384 XP002168263 & KISEICHUGAKU ZASSHI (1971), 20(2), 132-8 ,
- LINDSAY D S ET AL: "Examination of the activities of 43 chemotherapeutic agents against Neospora caninum tachyzoites in cultured cells" AMERICAN JOURNAL OF VETERINARY RESEARCH,XX,XX, Bd. 55, Nr. 7, Juli 1994 (1994-07), Seiten 976-981, XP002101367 ISSN: 0002-9645
- GOTTSTEIN B: "ZYSTENBILDENDE KOKZIDIEN: TOXOPLASMA, NEOSPORA, SARCOCYSTIS" SCHWEIZERISCHE MEDIZINISCHE WOCHENSCHRIFT,SCHWABE, BASEL,CH, Bd. 125, Nr. 18, 6. Mai 1995 (1995-05-06), Seiten 890-898, XP000961792 ISSN: 0036-7672
- PIPANO E: "LIVE VACCINES AGAINST HEMOPARASITIC DISEASES IN LIVESTOCK" VETERINARY PARASITOLOGY,NL,ELSEVIER SCIENCE, AMSTERDAM, Bd. 57, Nr. 1/03, 1. März 1995 (1995-03-01), Seiten 213-231, XP000610763 ISSN: 0304-4017
- HABERKORN A.: 'Chemotherapy of human and animal coccidioses: state and perspectives.' PARASITOL. RES. Bd. 82, Nr. 3, 1996, Seiten 193 - 199

## Beschreibung

Die vorliegende Erfindung betrifft Triazinonverbindungen zur Behandlung, insbesondere prophylaktischen Behandlung von Tieren, die mit Parasiten infiziert sind, welche zum Abortus führen oder Nervenkrankheiten verursachen. Insbesondere betrifft die vorliegende Erfindung diejenigen Triazinonverbindungen, die sich zur Behandlung von parasitischen Protozoen eignen, wie Coccidien, die zum Abortus führen oder Nervenkrankheiten verursachen. Ganz besonders betrifft die vorliegende Erfindung diejenigen Triazinonverbindungen, die sich zur Behandlung von Neospora-Infektionen eignen.

Triazinonverbindungen wie Triazindione, z.B. Diclazurile, sowie Triazintrione, z.B. Toltrazurile, wurden zur Behandlung von unterschiedlichen Säugetieren, Insekten und Fischen gegen Krankheiten, die von verschiedensten Protozoen verursacht werden, verwendet; siehe US-Patente 4,631,218; 4,933,341; 4,935,423; 5,114,938; 5,141,938; 5,188,832, 5,196,562, 5,256,631 und 5,464,837 oder auch EP A 170 316. Zu den Protozoen, die gegen diese Verbindungen empfindlich sind, zählen Parasiten, die die Eingeweide von Vögeln, Säugetieren und Insekten infizieren und sich in Form von Durchfall, Schwachwüchsigkeit, Übelkeit und Erbrechen äußern. Im Allgemeinen besteht die Wirkweise der Triazinone darin, die in Darm- und Eingeweidewandzellen vorhandenen Zwischenstadien des Parasiten anzugreifen, wodurch das endoplasmatische Reticulum, die den Zellkern umgebende Zone sowie die Mitochondrien des Parasiten anschwellen. Dies stört vermutlich die Teilungsfähigkeit des Zellkerns, wodurch die Schizonten und Mikrogamonten klein bleiben und jeweils nur einige wenige Merozoiten und Mikrogameten bilden. Das Endergebnis besteht darin, dass diese späteren Parasitenstadien die Fähigkeit, in neue Säugetierzellen einzudringen, verlieren, wodurch die Vermehrung des Parasiten im Wirt wirksam unterbunden wird.

Von besonderer Bedeutung sind bestimmte Protozoen, von denen seit den 70-er Jahren angenommen wird, dass sie Nervenkrankheiten verursachen und/oder zum Abortus bei Tieren führen. Die erfolgreiche Isolation und in-vitro-Kultivierung einiger solcher Protozoen erwies sich als schwierig. Zum Beispiel wurden Gehimflüssigkeit oder Rückenmarkflüssigkeit erst in den späten 80-er Jahren erfolgreich isoliert. Sobald feststand, dass Nervenkrankheiten von gehiminfizierenden Parasiten hervorgerufen werden können und zum Abortus führende Krankheiten von den fötusinfizierenden Parasiten hervorgerufen werden können, bestand ein Bedarf an effizient gegen Protozoen wirkenden Arzneistoffen, die die Blut-Gehirn-Schranke und die Plazentabarriere überwinden können, ohne schädigende Nebenwirkungen hervorzurufen. Nur sehr wenige Arzneistoffe sind fähig, die Blut-Gehirn-Schranke oder die Plazentaschranke von Tieren zu überwinden. Viele der aus dem Stand der Technik bekannten Arzneistoffe, die fähig sind, die Blut-Gehirn-Schranke und/oder die Plazentaschranke zu überwinden, um parasitische Infektionen des Gehirns wirksam zu behandeln, üben schädigende Nebenwirkungen aus, so dass sie nicht ohne hohes Risiko verwendet werden können. Bis jetzt sind daher keine wirksamen Arzneistoffe genehmigt worden, die eine wirksame Behandlung solcher Nerven- und zum Abortus führender Krankheiten darstellen. Es folgt nun eine kurze Beschreibung der durch Parasiten hervorgerufenen Krankheiten.

*Neospora caninum* ist ein neuer Parasit aus der Gruppe der Protozoen, welcher erstmals 1984 von BJERKAS et al. in einem norwegischen Hund beschrieben wurde. Natürliche Infektionen sind außer von Hund und Rind auch in Schaf, Ziege und Pferd nachgewiesen worden (Dubey and Rommel 1992, Dubey and Lindsay 1993). Experimentell gelang die Infektion außer bei Hund und Rind auch bei Fuchs, Katze, Schaf und Maus. Der Endwirt von *Neospora caninum* ist vermutlich der Hund (McAllister et al. 1998), ansonsten gibt es über den vollständigen Entwicklungszyklus noch keine detaillierten Untersuchungen.

Viele verschiedene Zellen wie Makrophagen, Neutrophile, Fibroblasten, Endothelzellen von Gefäßen, Myocyten, Epithelzellen der Nierentubuli, Hepatocyten und Nervenzellen können für *Neospora caninum* als Wirtszellen dienen. Jedoch erfolgt die Vermehrung über Tachyzoiten bevorzugt in Organellen wie Muskel und Nervenzellen. Daher treten die pathologischen Symptome nach einer natürlichen Infektion bevorzugt in diesen Geweben auf. So kommt es beim Hund nach einer natürlichen Infektion ab der 5. bis 6. Lebenswoche zu Krankheitssymptomen mit Anzeichen von Überempfindlichkeiten aufgrund von Nervenwurzelentzündungen und zunehmender Parese der Hinterbeine. Weitere histopathologische Befunde treten im Nervensystem, und zwar bevorzugt im Gehirn und Rückenmark auf. Hier dominieren ausgedehnte nichteitrige Entzündungen, Glia-Wucherungen und perivaskuläre Infiltrationen mit mononukleären Zellen (Makrophagen, Lymphocyten, wenige Plasmazellen) z.T. auch Eosinophile und Neutrophile. In der Muskulatur treten auch schon makroskopisch sichtbare nekrotisch-degenerative Veränderungen auf. Auffallend sind neben einer mehr oder weniger ausgeprägten Atrophie lange blasse Längsstreifen. Dies gilt insbesondere für die Hinterbeine. Histologisch stellen sich die Veränderungen als ausgeprägte Myositis mit leichten Nekrosen und nichteitrigen Gefäßentzündungen dar. Diese Veränderungen finden sich in abgeschwächter Form auch in der Muskulatur der Vorderbeine, des Zwerchfells und der Zungenmuskulatur. Hunde mit diesen Erscheinungen müssen im Alter von 5 bis 12 Wochen euthanasiert werden (Dubey et al. 1988). *Neospora*-Infektionen werden durch wiederholte transplazentale Übertragungen an die Folgegeneration weitergegeben. Dabei müssen in einem Wurf keineswegs alle Tiere erkranken. Bei einer experimentellen Übertragung auf 6 Hündinnen am 21. Tag der Trächtigkeit brachte 1 Tier 3 lebende Junge zur Welt, während die anderen 5 *N*. *caninum* positiven Foeten abortierten. In den 3 lebend geborenen Jungtieren ließ sich der Parasit nicht nachweisen (Cole et al. 1995).

Die erste historische Beschreibung im Rind erfolgte von Thilsted und Dubey (1989) im Himgewebe eines abortierten Foetus aus New Mexico. Weitere Isolationen aus dem Rind erfolgten in den USA (Conrad et al. 1993, Barr et al. 1993, Marsh et al. 1995), Japan (Yamane et al. 1996) und Schweden (Stenlund et al. 1996). In Kalifornien und Australien gelten *Neospora caninum* Infektionen als Hauptursache für Aborte in Rinderherden (Barr et al. 1990).

Wahrscheinlich werden die mit Abstand meisten *Neospora*-infizierten Kälber im Alter von 3 bis 9 Monaten abortiert. In diesen Foeten sind vor allem Tachyzoiten in größerer Zahl zu finden. Zysten wurden bisher erst bei geborenen Kälbern nachgewiesen. Infizierte Kälber sterben spätestens 3 bis 17 Tage nach der Geburt. Die Krankheitssymptome ähneln denjenigen beim Hund. Es treten Ataxien auf, die Gelenkreflexe sind stark abgeschwächt und es treten Paresen an den Hinterbeinen, z.T. an allen vier Beinen auf. Die histologischen Bilder ähneln denen, wie sie bei Hunden zu finden sind: Im Vordergrund stehen nichteitrige Meningitis und Myelitis. Im Gehirn finden sich- wie bei Hund und anderen Tieren mononukleäre Zellinfiltrate und Nekrosen insbesondere in perivaskulären Bereichen. Parasiten- vor allem Tachyzoiten bzw. Pseudozysten mit Tachyzoiten finden sich vor allem - meist jedoch nur in geringer Zahl- herdförmig im Nervengewebe, selten auch in Muskelzellen. Bemerkenswert und im klaren Kontrast zu *Toxoplasma* ist die Tatsache, dass *Neospora* von einem Muttertier mehrfach auf Nachkommen übertragen werden kann. Dies ist für Hunde und für Rinder nachgewiesen (Bjerkas et al. 1984, Dubey and Rommel 1992, Dubey et al. 1988).

Im Vergleich konnte bis jetzt kein Unterschied zwischen *N. caninum* Isolaten aus Hund und Rind gefunden werden, weder auf der Ebene der morphologischen Ultrastruktur, der Proteinanalyse noch nach molekularbiologischem Sequenzvergleich der rRNA oder ITS1 Sequenz (Holmdahl und Mattsson 1996).

### Verbreitung und wirtschaftliche Bedeutung

*N. caninum* konnte seit seiner Entdeckung 1984 inzwischen weltweit identifiziert werden (Review Dubey/Lindsay). Für Kalifornien wird der durch *N. caninum* bedingte Anteil an Aborten bei Kühen besonders hoch eingeschätzt. Von 468 abortierten Föten gingen 45,5 % auf eine *Neospora caninum* Infektion zurück (Dubey and Lindsay 1993). In der Schweiz konnte *Neospora*-spezifische DNA im Gehirn von 29 % abortierter Foeten nachgewiesen werden, demnach werden die jährlichen Verluste mit 10,2 Millionen Schweizer Franken geschätzt (Gottstein/Bern) sowie 100 Millionen Dollar in Australien (Johnson/Sydney). Bezüglich des Auftretens ergibt sich für Kalifornien eine Häufung im Winter von Dezember bis Februar. Entsprechende Befunde liegen für Neuseeland vor (Thornton et al. 1991). Hier werden die meisten *N. caninum* bedingten Aborte von Mai bis Juli registriert. Eine wirksame Methode zur Behandlung, insbesondere zur prophylaktischen Behandlung von Infektionen mit *N. caninum* ist bislang nicht bekannt geworden.

Bei der protozoenbedingten Myeloenzephalitis der Pferde (EPM) handelt es sich um eine Nervenerkrankung, die hauptsächlich bei jungen Pferden unter Stress auftritt (z.B. Vollblut-Rennpferden und Reinzucht-Hochleistungspferden) und es handelt sich daher um eine Krankheit mit beträchtlichen finanziellen Auswirkungen für die Pferdewirtschaft. EPM, die erstmals in den 70-er Jahren als Krankheit erkannt wurde, wurde erst 1991 von einem Pferd mit EPM kultiviert und erhielt den Namen *Sarcocystis neurona*. Im Jahr 1997 wurde eine Neospora spp., die jetzt den Namen *Neospora hugesi* trägt, aus dem Gehirn eines Pferdes mit EPM isoliert. Dementsprechend wird nun vorgeschlagen, dass EPM möglicherweise nur von diesem neuidentifizierten Organismus, nur von *Sarcocystis neurona* oder von einer Kombination dieser beiden Organismen verursacht wird. Am häufigsten führt EPM zum asymmetrischen Koordinationsversagen (Ataxie), zu Schwäche und zu Spastik. Die Krankheit kann beinahe alle Nervenkrankheiten vortäuschen. Sie kann in perakuter oder chronischer Form auftreten. Die chronische Form ist zu Beginn häufig heimtückisch, bis zu einem späten Zeitpunkt im Krankheitsverlauf schwer zu diagnostizieren und kann zum Tod führen. Bei den mildesten Fällen kann das einzige klinische Anzeichen eine unspezifische Lahmheit der Hüftgliedmaßen oder ein schwach ausgeprägtes Atemgeräusch sein. Bei den schwersten Fällen sind die Pferde schluck- oder stehunfähig. Es ist nun bekannt, dass bei den schwersten Fällen der Parasit, z.B. *S. neurona*, das Gehirn infiziert und dort erhebliche Schäden verursacht. Die klinischen Anzeichen von EPM werden durch direkte, von den Parasiten verursachten Nervenschäden (am Gehirn und Rückenmark) sowie von Gehirnschäden aufgrund Infiltration von Entzündungszellen, Ödem sowie mit Merozoiten und Meronten koexistierendem Absterben der Nerven im Zentralnervensystem (ZNS) verursacht. Zur Zeit existiert keine wirksame Prophylaxe zur Kontrolle von EPM. Die Kombination der Humanarzneistoffe Trimethoprim und Sulphonamid wurde verwendet. Die Behandlung ist jedoch teuer und erfordert viele Wiederholungsdosen.

Ein weiterer zur Gruppe der Coccidien gehörender Parasit, nämlich *Toxoplasma gondii*, ist seit geraumer Zeit bekannt und wurde zum ersten Mal aus den Eingeweiden und dem Muskelgewebe der Katze isoliert. Der Endwirt dieses Parasiten ist die Katze, und diese kann den Organismus über lange Zeit beherbergen, während der sich die Oocysten auf andere Tiere, darunter Rinder, Schafe, Schweine und Menschen, ausbreiten. Die Infektion von Schafen, Rindern und Menschen wurde mit zum Abortus führenden Krankheiten und mit angeborenen Krankheiten, die hauptsächlich das Zentralnervensystem angreifen, in Zusammenhang gebracht. In jüngster Zeit wurde es auch mit dem Abortus und Missbildungen an Kätzchen, die von einer infizierten Mutter stammen, die vor der Infektion während der Trächtigkeit seronegativ war, in Zusammenhang gebracht. Nichtkatzenartige Wirte wie Rinder, Schafe, Schweine und Menschen produzieren keine Oocysten, es kommt jedoch zur Entwicklung von Tachyzoiten und Bradyzoiten und gegebenenfalls zur Invasion von Muskeln und Gehirn durch diese, wobei die Tachyzoiten und Bradyzoiten die klinischen Anzeichen der Krankheit, nämlich neurologische Symptome sowie Abortus mit Missbildungen des. Fötus, verursachen. Es wurde berichtet, dass 60 % aller Katzen serologisch positiv für *T. gondii* sind. Wiederum existiert keine Behandlung, insbesondere prophylaktische Behandlung für Toxoplasmose.

Die Verwendung von Triazinonverbindungen wie Diclazuril, Toltrazuril oder Toltrazuril-Sulphon (seit kurzer Zeit unter der neuen Bezeichnung "Ponazuril" bekannt) zur Behandlung, insbesondere prophylaktischen. Behandlung, ohne nichtakzeptable Nebenwirkungen, von Tieren, die einem Infektionsrisiko mit Coccidien, insbesondere aus der Familie Sarcocystidae, ausgesetzt sind, ist aus Literaturangaben des Standes der Technik, darunter auch den eingangs genannten Literaturangaben, nicht bekannt bzw. wird in diesen nicht gelehrt. Es stellte sich daher die Aufgabe eine effiziente Behandlung, insbesondere prophylaktische Behandlung für Tiere bereitzustellen, die einem Infektionsrisiko mit den genannten Parasiten ausgesetzt sind.

Es wurde nun überraschenderweise gefunden, dass man durch Verwendung von Triazinonen zur Behandlung, insbesondere zur prophylaktischen Behandlung, einen durchgreifenden Schutz vor Infektionen mit parasitären Protozoen erreicht.

Die vorliegende Erfindung betrifft demnach
1. Verwendung von Toltrazuril oder Ponazuril zur Herstellung von Mitteln zur prophylaktischen Behandlung von Tieren gegen *Neospora caninum*.
2. Verwendung von Toltrazuril oder Ponazuril zur Herstellung von Mitteln zur Anwendung in Kombination mit Lebend- oder Totvakzinen bei der Behandlung von Tieren gegen parasitäre Protozoen.
3. Mittel enthaltend einen Triazinon-Wirkstoff ausgewählt aus Ponazuril und Toltrazuril sowie eine Lebend- oder Totvaccine als Kombinationspräparat gegen parasitäre Protozoen.

Beispiele für Tiere, die erfindungemäß behandelt werden können, sind Pferde, Rinder, Katzen, Hunde, Schweine, Schafe, Vögel, Insekten und Menschen.

Bei den Parasiten, welche die Infektion oder Krankheit verursachen, handelt es sich um Coccidien der Familie Sarcocystidae, die sich als Nervenkrankheiten oder zum Abortus führende Krankheiten manifestieren können. Beispiele zur Veranschaulichung, die jedoch nicht als Beschränkung aufzufassen sind, können aus der Gruppe Sarcocystis spp., Neospora spp. und Toxoplasma spp., ausgewählt werden. Typischerweise werden die Sarcocystidae ausgewählt aus der Gruppe *S neurona, N. hugesi, N. caninum* und *T. gondii,* insbesondere aus der Gruppe *Neospora spp*.. Zu den Protozoeninfektionen oder -krankheiten zählen EPM, Neosporosis und Toxoplasmosis, sie sind jedoch nicht hierauf beschränkt.

Bei der Durchführung der Erfindung führt die Behandlung, insbesondere prophylaktische Behandlung der Tiere gegen die von den hier beschriebenen Protozoen verursachten parasitären Infektionen oder Krankheiten zur Verhinderung der Symptome und der damit verbundenen Krankheiten. Im Allgemeinen zählen zu den Symptomen dieser Krankheiten Lahmheit, Ataxie, Lähmung, Abortus, schwächliche Neugeborene sowie andere verwandte Störungen. Typischerweise dauert die Behandlung ungefähr 1 bis 30 Tage, vorzugsweise ungefähr 1 bis 20, besonders bevorzugt 1 bis 10 und ganz besonders bevorzugt 1 bis 6 Tage. Natürlich kann für die Behandlung, insbesondere prophylaktische Behandlung das Behandlungsschema einmal pro Tag, zweimal oder mehrmals pro Tag, einmal alle zwei Tage oder sogar einmal pro Woche betragen, je nach Umständen wie Art des krankheitserregenden Parasiten.

Bevorzugte Dosierungen liegen bei 1-500 mg Wirkstoff pro kg Körpergewicht des zu behandelnden Tieres, besonders bevorzugt sind Dosen von 10 bis 200 mg/kg und ganz besonders bevorzugt sind Dosen von 20-150 mg/kg.

Ohne sich auf eine bestimmte Theorie der Erfindung festlegen zu wollen, wird angenommen, dass der unerwartete Erfolg der hier beschriebenen Behandlung, insbesondere prophylaktischen Behandlung, auf der Fähigkeit der Triazinonverbindungen, die Blut-Gehirn-Schranke oder die Plazentaschranke zu überwinden, beruht. Es wird angenommen, dass die Verbindungen der vorliegenden Erfindung die Blut-Gehirn-Schranke leicht überwinden und auch fähig sind, in die Plazenta einzudringen und die Protozoen in situ im Gehirn und in der Rückenmarkflüssigkeit im Rückenmark abtöten.

Bis Dato waren keine kostengünstigen, einfach zu verabreichenden Arzneistoffe für den wirksamen Schutz gegen diese Krankheiten, die keine bedenklichen Nebenwirkungen wie Toxizität oder Mutagenität bei Tieren aufweisen, verfügbar.

Erfindungsgemäß verwendet werden die Verbindungen 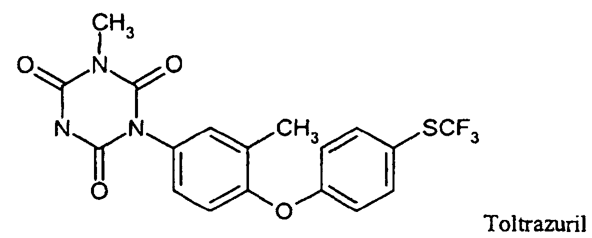 und 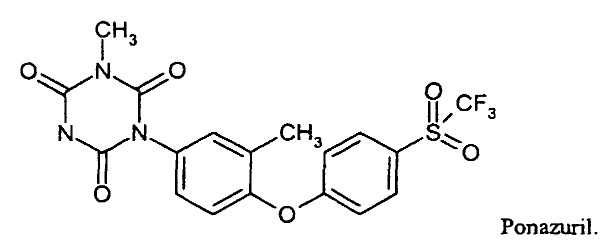
(a) Triazintrione können nach einem allgemeinen Verfahren erhalten werden, wenn man Verbindungen der Formel II 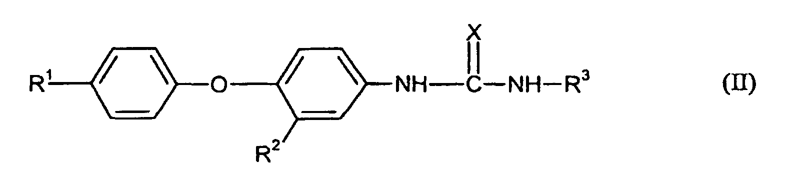 in der
   - R¹, R², R³ und X: die entsprechenden Bedeutungen aufweisen, mit einem substituierten Carbonsäure-isocyanat der Formel III 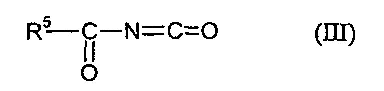
   in der
   - R⁵: ein Halogenatom, eine Alkoxygruppe oder eine Aryloxygruppe bedeutet, umsetzt, und die so gebildeten substituierten 1,3,5-Triazinderivate der Formel IV 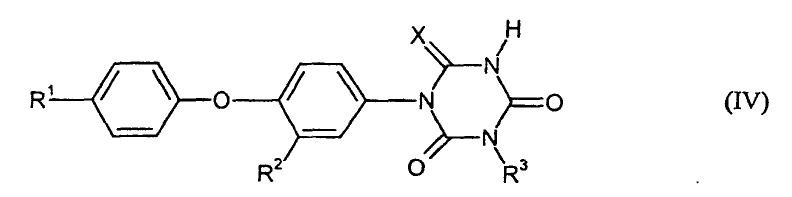
   in der
   - R¹, R², R³ und X: die entsprechenden Bedeutungen aufweisen, gegebenenfalls isoliert werden und gegebenenfalls mit einer Verbindung der Formel V

   A - Z (V)
   in der
   - A: Alkyl, Alkenyl oder Alkinyl darstellt und
   - Z: Halogen darstellt, umgesetzt werden;
(b) Triazintrionverbindungen können weiterhin erhalten werden, wenn man Verbindungen der Formel II, in der R¹, R², R³ und X die oben angegebenen Bedeutungen aufweisen, mit Bis(chlorCarbonsäure)aminen der Formel VI 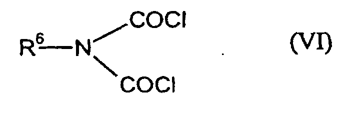 in der
   - R⁶: Alkyl darstellt, gegebenenfalls in der Gegenwart von Säureakzeptoren umsetzt,
(c) Triazintrionverbindungen, in denen die Substituenten R², R³ und R⁴ sowie X die entsprechenden Bedeutungen aufweisen und R¹ Halogenalkylsulfmyl oder Halogenalkylsulfonyl bedeutet, können erhalten werden, indem man Verbindungen der Formel 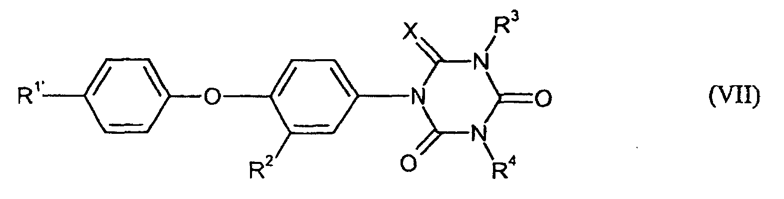 in der
   - R², R³ und R⁴: die entsprechenden Bedeutungen aufweisen und
   - R^{1'}: Halogenalkylthio darstellt,
   mit der geeigneten Menge eines geeigneten Oxidationsmittels umsetzt.

Verwendet man bei der Verfahrensvariante (a) N-[3-Chlor-4-(4'-trifluormethylthio-phenoxy)-phenyl]-N'-methylharnstoff und Chlorcarbonsäureisocyanat, so lässt sich der Reaktionsablauf durch die folgende Gleichung ausdrücken:

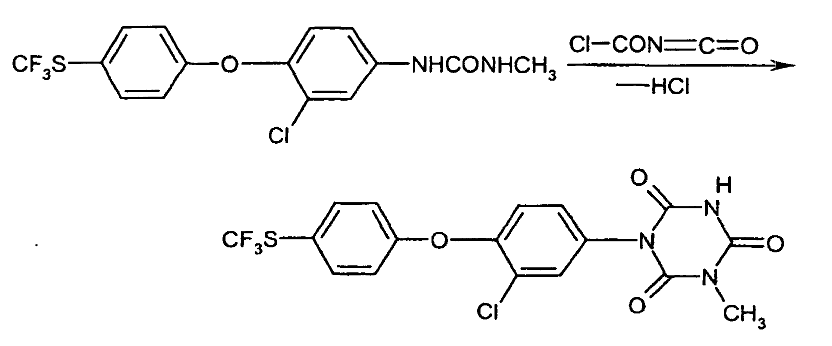

Verwendet man in der Verfahrensvariante (b) N-[3-Ethoxy-4-(4'-trifluormethyl-thio-phenoxy)-phenyl]-thioharnstoff und N-Methyl-bis-(chlorcarbonsäure)amin als Ausgangssubstanzen, so lässt sich der Verfahrensablauf durch die folgende Gleichung darstellen:

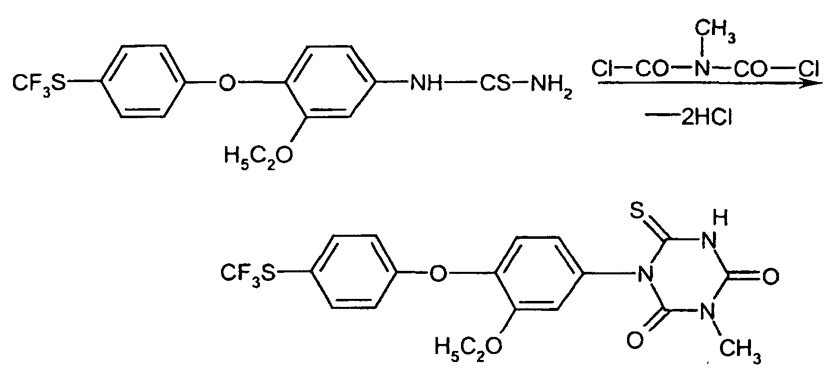

Die gemäß der Verfahrensvariante (a) oder (b) erhaltenen Verbindungen, in denen R¹ Halogenalkylthio und X O bedeuten, können gemäß der Verfahrensvariante (c) zu den entsprechenden Halogenalkylsulfinyl- oder Halogenalkylsulfonyl-derivaten oxidiert werden. Verwendet man als Oxidationsmittel Wasserstoffperoxid, so lässt sich der Verfahrensablauf durch die folgende Gleichung darstellen:

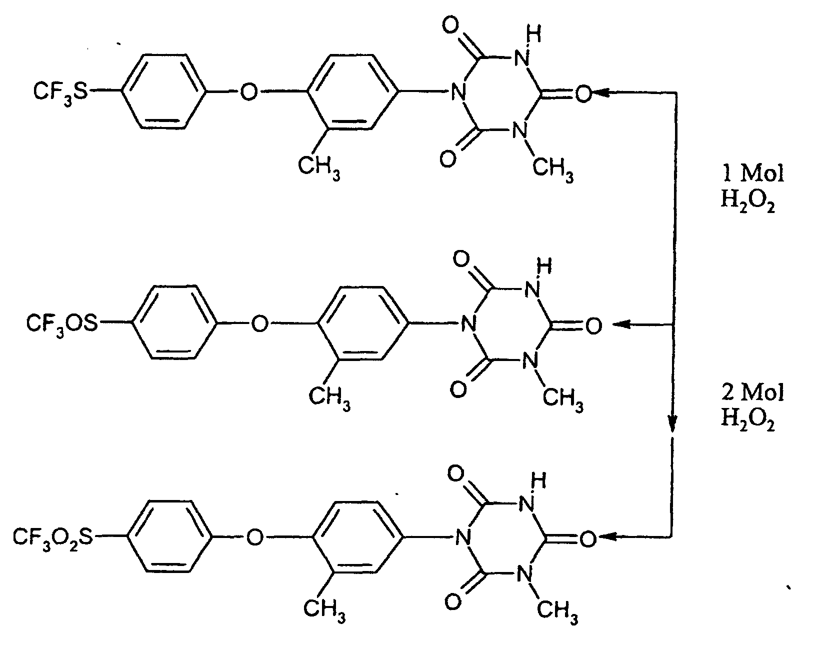

In den Formeln II, IV, V, VI und VII handelt es sich bei dem bei R⁶ oder A definierten Alkyl um geradkettiges oder verzweigtes Alkyl mit vorzugsweise 1 bis 6, insbesondere 1 bis 4 Kohlenstoffatomen. Beispielsweise können gegebenenfalls substituiertes Methyl, Ethyl, n- und i-Propyl sowie n-, i- und t-Butyl genannt werden.

In den Formeln II, IV, V und VII bedeutet für A definiertes Alkenyl geradkettiges oder verzweigtes Alkenyl mit vorzugsweise 2 bis 6, insbesondere 2 bis 4, Kohlenstoffatomen. Beispielsweise können gegebenenfalls substituiertes Ethenyl, Propen-1-yl, Propen-2-yl und Buten-3-yl genannt werden.

In den Formeln II, IV, V und VII bedeutet für A definiertes Alkinyl geradkettiges oder verzweigtes Alkinyl mit vorzugsweise 2 bis 6, insbesondere 2 bis 4, Kohlenstoffatomen. Beispielsweise können gegebenenfalls substituiertes Ethinyl, Propin-1-yl, Propin-2-yl und Butin-3-yl genannt werden.

In den Formeln II, III, IV und VII bedeutet für R⁵ definiertes Alkoxy geradkettiges oder verzweigtes Alkoxy mit vorzugsweise 1 bis 6, insbesondere 1 bis 4, Kohlenstoffatomen. Beispielsweise können gegebenenfalls substituiertes Methoxy, Ethoxy, n- und i-Propoxy sowie n- und i-Butoxy genannt werden.

In den Formeln II, III, IV, V und VII bedeutet für R⁵ oder Z definiertes Halogen vorzugsweise Fluor, Chlor, Brom und Iod, insbesondere Chlor und Brom.

In der Formel III bedeutet für R⁵ definiertes Aryloxy vorzugsweise monocyclisches carbocyclisches Aryloxy oder bicyclisches carbocyclisches Aryloxy, insbesondere Phenoxy.

In der Formel III bedeutet Aryloxy in der Bedeutung von R⁵ vorzugsweise Phenoxy. Die meisten der substituierten Harnstoffe oder Thiohamstoffe der Formel II, die als Ausgangssubstanzen verwendet werden, waren bis Dato unbekannt, können jedoch mit als solchen bekannten Verfahren leicht hergestellt werden, und zwar dadurch, dass man (a) entweder substituierte 4-Aminodiphenylether mit den entsprechenden substituierten Isocyanaten oder Isothiocyanaten in einem inerten Lösungsmittel bei Temperaturen zwischen 0°C und 100°C umsetzt oder, in umgekehrter Reihenfolge, (b) Ammoniak oder substituierte Amine und die entsprechenden substituierten Isocyanat- oder 4-Isothiocyanat-diphenylether miteinander unter den gleichen Bedingungen umsetzt, oder dadurch, dass man (c) substituierte 4-Hydroxyphenyl-harnstoffe oder -thioharnstoffe mit aktiven halogensubstituierten aromatischen Verbindungen in aprotischen Lösungsmitteln wie Dimethylsulphoxid, Dimethylformamid oder Hexamethylphosphorsäuretriamid in der Gegenwart von Basen wie Natriumhydrid, Kaliumhydroxid, Kaliumcarbonat und anderen bei Temperaturen zwischen 20°C und 150°C kondensiert.

Bei geeigneter Auswahl des Lösungsmittels kristallisieren die Reaktionsprodukte im Allgemeinen beim Abkühlen der Lösung aus. Die Harnstoffe können jedoch auch alternativ aus Aminen und Isocyanaten nach der folgenden Literaturstelle hergestellt werden: Methoden der Org. Chemie (Houben-Weyl) IV. Ausgabe, Band VIII, Seite 157-158.

Einige der Bis(chlorcarbonsäure)-amine der allgemeinen Formel VI, die gemäß der vorliegenden Erfindung im Verfahren (b) verwendet werden können, sind bereits bekannt (vgl. Artikel in Synthesis 1970, Seite 542-543), und diejenigen, die bis jetzt noch nicht bekannt sind, lassen sich auf analoge Weise aus cyclischen Diacyldisulfiden mittels Chlorierung in inerten organischen Lösungsmitteln, vorzugsweise Kohlenstofftetrachlorid, herstellen.

Als mögliche Verdünnungsmittel für die Umsetzung der Harnstoffe oder Thioharnstoffe der Formel II sowohl mit den Carbonsäureisocyanaten der Formel III (Verfahrensvariante a) als auch mit den Bis(chlor-carbonsäure)-aminen der Formel VI (Verfahrensvariante b) sowie für die Umsetzung der 1,3,5-Triazinderivate der Formel IV mit Verbindungen der Formel A-Z sind alle diejenigen organischen Lösungsmittel geeignet, die gegenüber diesen Reaktionen inert sind.

Zu diesen zählen außer Pyridin vorzugsweise aromatische Kohlenwasserstoffe wie Benzol, Toluol und Xylol, halogenierte aromatische Kohlenwasserstoffe wie Chlorbenzol und Dichlorbenzol, sowie Ether wie Tetrahydrofuran und Dioxan.

Die Chlorwasserstoffsäure, die sich während der Reaktion bilden kann, entweicht in Form eines Gases oder kann mittels organischer oder inorganischer Säureakzeptoren gebunden werden. Zu diesen Säureakzeptoren zählen vorzugsweise tertiäre organische Basen wie Trialkylamine, zum Beispiel Triethylamin, aromatische N-Hetero(mono- oder bi)cyclische Amine wie mono- oder bicyclische Pyridinaza-cycloalkylamine wie Diazabicyclononen, Diazabicycloundecen und viele andere mehr, oder inorganische Basen wie Alkalicarbonate, -oxide oder -hydroxide oder Erdalkalicarbonate, -oxide oder -hydroxide.

Die Reaktionstemperaturen für die oben genannten Reaktionsschritte können innerhalb weiter Grenzen schwanken. Im Allgemeinen wird die Reaktion zwischen ungefähr 0°C und ungefähr 150°C, vorzugsweise zwischen ungefähr 20°C und ungefähr 100°C, durchgeführt.

Die oben genannten Reaktionsschritte können unter Normaldruck oder unter erhöhtem Druck vorgenommen werden. Im Allgemeinen arbeitet man unter Normaldruck.

Mögliche Oxidationsmittel für die Umsetzung nach Verfahrensvariante (c) der Trifluormethylthiotriazonen zu den entsprechenden Sulfinyl- oder Sulfonylverbindungen sind jeweils H₂O₂/Eisessig, H₂O₂/Essigsäureanhydrid, H₂O₂/Methanol, Persäuren wie zum Beispiel m-Chlorperbenzoesäure, sowie Chromsäure, Kaliumpermanganat, Natriumperiodat, Cer(IV)ammoniumnitrat sowie Salpetersäure.

Eine Triazinonverbindung kann zum Beispiel durch Umsetzen mit einer anorganischen oder organischen Base in ein entsprechendes Additionssalz umgewandelt werden.

Bei der Durchführung der Erfindung kann die Triazinonverbindung zur Verabreichung an Tiere auf eine beliebige Weise formuliert werden. Formulierungen, die sich für die orale Verabreichung, die in diesem Zusammenhang bevorzugt ist, eignen, können Lösungen, Suspensionen, Tabletten, Kapseln, Gele, Pasten, Boli oder Präparate in Form von Pulvern, Granulaten oder Pellets sein. Zu weiteren Verabreichungsmöglichkeiten zählen die parenterale, topische, intramuskuläre und intramucosale Verabreichung oder andere Verabreichungswege, die dem Fachmann bekannt sind. Die topische Verabreichung in Form eines Mittels zum Aufgießen (pour-on) ist ebenfalls bevorzugt.

Eine besonders effektive Anwendung ist die Kombination von Toltrazuril oder Ponazuril mit Lebend- oder Totvaccinen gegen parasitäre Protozoen, insbesondere gegen Neospora spp. Hierbei können auch wirkungssteigemde Effekte beobachtet werden.

Mittel und Formulierungen werden durch Mischen der Komponenten in geeigneten Apparaturen wie Rührkesseln und anderen geeigneten Geräte hergestellt.

Die Erfindung wird durch die folgenden Beispiele genauer beschrieben:

### Beispiele

### Neospora caninum Infektionen

Grundlage einer Diagnose von *Neospora caninum* und die Abgrenzung zu *Toxoplasma gondii* sind klinische, mikroskopische, immunhistochemische und molekularbiologische Parameter. Klinisch tritt eine Lähmung von Extremitäten und eine wiederholte transplazentare Übertragung auf. Mikroskopisch können Tachyzoiten und Zysten in Muskulatur und Nervengewebe auftreten. Zysten mit einer ausgeprägten dickwandigen Zystenwand treten nur in geringer Zahl und ausschließlich im Nervengewebe auf. Im indirekten Immun-Fluoreszenztest (IFAT) unter Verwendung von in Zellkultur erzeugten Tachyzoiten, gelten Titer ab 1:200 als spezifisch. Bei bereits vorliegenden klinischen Symptomen können Titer bis 1:20.000 auftreten. Molekularbiologisch gelingt eine schnelle und eindeutige Identifikation von *Neospora caninum* durch eine in vitro Amplifikation der ITS1-Region (internal transcriber spacer 1) (Holmdale and Mattsson 1996).

### Beschreibung des in vitro Testsystems

### Kultivierung von VERO-Wirtszellen

*Neospora caninum* ist ein obligat intrazellulärer Parasit. VERO-Zellen (Nierenzellen einer afrikanischen grünen Meerkatze, ATCC Nr.: CCL 81 Vero) dienten als Hilfsmittel, den Parasiten unter standardisierbaren und definierbaren Bedingungen zur Reproduktion zu bringen. Vero-Zellen wurden in folgendem Medium kultiviert: 87 % RPMI 1648 (ICN, 12-602-54) 10 % FCS (foetales Kälberserum, ICN, 29-101-49) 1 % 200 mM L-Glutamin (ICN, 15-801-13) 1 % Natriumbicarbonat (ICN, 16-883-49) 1 % Penicillin/Streptomycin (ICN, 16-700-49). Die Haltung und Passage nicht infizierter Kulturen erfolgte in Gewebekulturflaschen von 25 cm² (Falcon, B 769031) und 75 cm² (Falcon, B 769051). Vero-Zellen wurden bei 37°C unter 5 % CO₂-Atmosphäre in einem CO₂-Inkubator (Heraeus) bis zur Ausbildung eines Monolayer-Zellrasens kultiviert.

### Kultivierung von ED-Zellen

ED-Zellen (dermale Pferdezellen, ATTC Nr. CCL 57) wurden in folgendem Medium kultiviert: 87 % EMEM (ICN, 12-106-54) 10 % FCS (foetales Kälberserum, ICN, 29-101-49), 1 % 200 mM L-Glutamin (ICN, 15-801-13), 1 % NEA (Nichtessentielle Aminosäuren, Gibco, 11140-035), 1 % Penicillin/Streptomycin (ICN, 16-700-49). Die Haltung und Passage nicht infizierter Kulturen erfolgte in Gewebekulturflaschen von 25 cm² (Falcon, B 769031) und 75 cm² (Falcon, B 769051). ED-Zellen wurden bei 37°C ohne CO₂-Atmosphäre in einem Inkubator (Hereus) bis zur Ausbildung eines Monolayer-Zellrasens kultiviert.

### Passagieren von Zellen

Wirtszellen wurden passagiert, d.h. auf neue Zellkulturgefäße verteilt, wenn der Zellrasen einer Kultur in den Kulturflaschen dicht geschlossen war. Bei einem Gehalt von 10 % FCS im Kulturmedium erfolgte dies in der Regel zweimal pro Woche. Zunächst wurde das Kultur-Medium dekantiert, der Zellrasen mit 5 ml Trypsin-EDTA (ICN, 16-891-49) gespült und mit weiteren 5 ml Trypsin-EDTA 5-10 min bei 37°C im CO₂-Brutschrank inkubiert, bis sich die Zellen vom Boden gelöst hatten. Die Trypsin-EDTA-Zellsuspension wurde zusammen mit 1-2 ml vorgewärmtem FCS bei 1500 UpM (Varifuge 3.0, Heraeus) 5 min zentrifugiert. Der Überstand wurde verworfen und das Pellet in 15 ml Medium gelöst (92 % RPMI 1640, 5 % FCS, 1 % L-Glutamin, 1 % Natriumbikarbonat, 1 % Penicillin/Streptomycin). Pro Gewebekulturflasche wurden 3 neue Flaschen mit jeweils 5 ml Zellsuspension angeimpft, d.h. die Teilungsrate betrug 1:3.

### Gefrierkonservierung von Zellen im flüssigen Stickstoff

Zellen aus Kulturflaschen wurden entweder im C541-Medium (50 % RPMI 1640, 40 % foetalem Kälberserum (FCS), 10 % Dimethylsulfoxid (DMSO, Merck 9578) oder im C2-Medium (86 % RPMI 1640, 10 % DMSO, 2 % FCS, 1 % L-Glutamin, 1 % Streptomycin/Penicillin) eingefroren. Zuvor wurden die Zellen wie beschrieben (s. oben) trypsiniert, die abgelösten Zellen zentrifugiert und in 3 ml C541-Einfriermedium oder C2-Medium resuspendiert und in 2 ml-Kryoröhrchen überführt. Die Endlagerung erfolgte im füssigen Stickstoff bei -196°C, dort sind Zellen unbegrenzt haltbar. Vor der Endlagerung im flüssigen Stickstoff wurden die Kryoröhrchen mit den Zellinien in einer Styroporbox von 1 cm Wanddicke langsam auf -80°C abgekühlt. Die Styroporbox ermöglicht eine kontinuierliche Abkühlgeschwindigkeit von 1-2°C pro Minute, bei der die Zellen Gelegenheit haben über Osmose ihr intrazelluläres Wasser zu verlieren. Dies ist für die Vitalität der Zellen von entscheidender Bedeutung.

### Auftauen gefrierkonservierter Zellen

Der Auftauvorgang der Kryoröhrchen aus flüssigem Stickstoff erfolgte so schnell wie möglich in einem 37°C warmen Wasserbad. Die Zellsuspension wurde in 10 ml Medium pipettiert und anschließend auf zwei 50 ml-Gewebekulturflaschen gleichmäßig verteilt. Die Kultivierung erfolgte bei 37°C und 5 % CO₂. Nach 24 h wurde das Zellkulturmedium gewechselt, um das im Einfriermedium enthaltene DMSO zu entfernen.

### Infektion von Zellkulturen mit Neospora caninum

Für die Infektion von Zellkulturen wurden folgende *Neospora caninum* Isolate verwendet: NC-1 (Isolat aus dem Hund DUBEY (1988) und NC Swe B-1/ 9. Passage (Isolat aus dem Rind; National Veterinary Institute Uppsala, Schweden). Als Infektionsmaterial dienten infizierte Zellkulturen aus dem Stickstofflager (s. oben) oder gereinigte Tachyzoiten infizierter Kulturen (s. unten).

### Aufreinigung von Tachyzoiten aus Zellkulturen mittels Sephadex

Die Reinigung von *Neospora caninum* aus infizierten VERO-oder ED-Monolayern erfolgte unter sterilen Bedingungen. Zunächst wurden infizierte Zellkulturen mit einem Zellschaber (Tec No Mara, 3010) vom Flaschenboden gelöst und mit einer 23-er Kanüle (Luer 23 Gx1, 0,6x25 mm) in eine 10 ml Einwegspritze gezogen. Bei diesem Vorgang wurden Wirtszellen und darin enthaltene Gewebezysten mechanisch zerstört. Die Zellsuspension wurde anschließend bei 1500 UpM (Varifuge 3.0, Heraeus) 7 min lang zentrifugiert, der Überstand verworfen und das Proben-Pellet in genau 2,5 ml physiologischem Phosphat-Puffer (PBS: 1 mM P04, 12 mM NaCl, 0,87 mM KCL, pH 7.4) resuspendiert. Die weitere Aufreinigung erfolgte über eine Sephadex-Säule (PD-10™/Sephadex G-25 M, Pharmacia Biotech, 17-0851). Die Säule wurde zunächst mit 25 ml PBS equilibriert, das Probenvolumen in 2,5 ml PBS aufgetragen und danach mit 5 ml PBS eluiert. Tachyzoiten laufen schnell durch die Säule und befinden sich in den ersten 3 ml des Eluates. Hochmolekulare Zelltrümmer und Membranen verlassen zeitlich verzögert die Sephadex-Säule. Um unerwünschte Zellorganellen und freie Wirtszell-DNA zu entfernen wurde die Probe bei 1500 UpM 7 min lang zentrifugiert, der Überstand verworfen und das Pellet 3mal in 40ml PBS gespült.

### Testen von Substanzen an infizierten Neospora caninum Zellkulturen

Das Testen von Substanzen fand in 96-well-Platten (Falcon 3872) statt, weil in diesem System nur geringe Mengen an Ausgangssubstanz (ca. 1 mg) benötigt werden. Auf den Zellkulturplatten wurde zunächst ein Zellkultur-Monolayer der Wirtszellen (Vero oder ED) angelegt. Hierfür wurden zwei 50 ml Gewebekulturflaschen (insgesamt 50 cm² Zellkulturfläche) mit einem ausgebildeten nicht infizierten Monolayer benötigt. Der Zellrasen dieser Kultur wurde mit 5 ml Trypsin-EDTA (Gibco, 45300-019) im CO₂-Brutschrank bei 37°C abgelöst. Nach 10 min. Inkubationszeit hatten sich die Zellen zum größten Teil abgelöst. Mit einer 5 ml-Pipette wurde die Zellsuspension in ein 50 ml-Zentrifugenröhrchen (Greiner, B769331) überführt, indem ca 1 ml angewärmtes foetales Kälberserum vorgelegt wurde. Nach 5 min Zentrifugation bei 1500 UpM (Varifuge 3.0, Heraeus) wurde der Überstand verworfen und das Zell-Pellet in 100 ml RPMI-Medium (95 % RPMI 1640, 2 % FCS, 1 % L-Glutamin, 1 % Natriumbicarbonat, 1 % Penicillin/Streptomycin) resuspendiert. Von dieser Zellsuspension wurden 150 µl pro well einer 96-well Platte einpipettiert. 100 ml Medium reichen für die Beschichtung von 6-Mikrowellplatten. Beschichtete Zellkultur-Platten wurden im Brutschrank bei 37°C unter 5 % CO₂ für 24 h kultiviert. Danach erfolgte eine Infektion mit gereinigten *Neospora caninum* Tachyzoiten bei einer Konzentration von 48.000 Tachyzoiten pro well und einer weiteren Inkubation bei 37°C und 5 % CO₂ für 24 h. Die zu testenden Substanzen wurden in 1,5 ml Eppendorfreaktionsgefäße eingewogen, wobei die eingewogenen Menge bei 0,5-1,5 mg lag. Anschließend wurde pro mg Substanz 1 ml DMSO zupipettiert, was einer Verdünnung von 1x10⁻³ g/ml entspricht. Das Medium zur Behandlung, indem die weiteren Verdünnungsreihen erfolgten, bestand aus 87 % RPMI 1640, 10 % FCS, 1 % L-Glutamin, 1 % Natriumbicarbonat, 1 % Penicillin/Streptomycin. Beim ersten Screening wurden die Konzentrationen 10 ppm, 25 ppm und 50 ppm eingesetzt. 24 h nach der Infektion durch *Neospora caninum* wurden die verdünnten Präparate in einem Volumen von 150 µl/Well auf die Zellktilturplatten übertragen. In der ersten Reihe wurde unbehandeltes Medium verwendet, diese Reihe enthielt sowohl die infizierte Kontrolle als auch die nicht infizierte Kontrolle. Danach erfolgte eine Inkubation der Zellplatten für 5 Tage bei 37°C und 5 % CO₂. Innerhalb dieser Zeit vermehren sich die Tachyzoiten innerhalb der Wirtszellen und führen dadurch zur Zerstörung des Monolayers. Bei voller Wirksamkeit einer Substanz werden die Tachyzoiten abgetötet und der Monolayer bleibt erhalten. Ein intakter Monolayer kann durch einen Protein-Bindungs-Test (Lebendfärbung) nachgewiesen werden. Eine Methode hierfür ist die Färbung mit 0,25 % Crystal Violet (Sigma C 3886). Vor Färbung wurden die Testplatten mit 100 µl PBS gespült und mit 100 µl Methanol fixiert. Die mikroskopische Auswertung erfolgte 4 Tage nach Behandlungsbeginn und 5 Tage nach der Infektion bei einer Vergrößerung von 25 x 10 in einem Umkehrmikroskop nach folgendem Bewertungsschema:

| **Bewertung** | **Optischer Eindruck** |
|---|---|
| 0 = keine Wirkung | Monolayer vollständig zerstört |
| 1 = schwache Wirkung | Monolayer teilweise zerstört, Parasitennester zu erkennen |
| 2 = volle Wirkung | Monolayer unzerstört, keine Tachyzoiten zu erkennen |
| T = cytotoxisch | Zellen abgestorben, abgekugelt |

**Tabelle 1:**

| **Wirksamkeit von Toltrazuril gegen** ***Neospora caninum*** **in VERO Zellen** | | | |
|---|---|---|---|
| **Substanz** | **50 ppm** | **25 ppm** | **10 ppm** |
| **Infizierte Kontrolle** | **0** | **0** | **0** |
| **Toltrazuril reiner Wirkstoff** | **2** | **2** | **1** |
| **Toltrazuril 2,5 % Lösung*** | **2** | **2** | **1** |

| | | | |
|---|---|---|---|
| * 100 ml Lösung enthalten: 2,50 g Toltrazuril 30,00 g Triethanolamine 80,70 g Polyethylene glycol, die Komponenten werden einfach gemischt. | | | |

| **Bewertung** | **Optischer Eindruck** |
|---|---|
| 0 = keine Wirkung | Monolayer vollständig zerstört |
| 1 = schwache Wirkung | Monolayer teilweise zerstört, Parasitennester zu erkennen |
| 2 = volle Wirkung | Monolayer unzerstört, keine Tachyzoiten zu erkennen |
| T = cytotoxisch | Zellen abgestorben, abgekugelt |

### II. In vivo Wirksamkeit

Für die in vivo-Wirksamkeit von Substanzen liegen bis jetzt nur sehr wenige Angaben vor, da adäquate in vivo-Testsysteme noch entwickelt werden müssen. Bei experimentell infizierten Mäusen erwies sich Sulfadiazin (über das Trinkwasser verabreicht) nur als wirksam, wenn die Behandlung prophylaktisch, d.h. vor der Infektion begann. Dann wurden klinische Symptome verhindert, ohne dass es zu einer parasitologischen Heilung kam. Ein späterer Behandlungsbeginn war erfolglos (Bjerkas et al. 1984, Dubey et al. 1988, Lindsay and Dubey 1989, Lindsay and Dubey 1990). Beim Hund hat die Behandlung mit Sulfadiazin und Clindamycin nur dann eine Erfolgsschance, wenn sie sehr frühzeitig bei dem ersten Auftreten klinischer Symptome infolge der Nervenwurzelentzündungen beginnt.

### Beschreibung des Testmodells in der Maus

Die Versuchsplanung und Durchführung erfolgten im Auftrag der BAYER AG durch Dr. Simone Eperon & Prof. Bruno Gottstein am Institut für Parasitologie der Veterinärmedizinischen und der Medizinischen Fakultät der Universität Bern (Eperon et al. 1999, Parasite Immunology 21:225-236.)

### Mäuse

Weibliche Wt C57BL/6-Mäuse. Alter der Tiere zum Zeitpunkt der Behandlung: 7 ½ Wochen.

### Neospora caninum Tachvzoiten

*Neospora caninum* Tachyzoiten wurden über VERO Zellen passagiert, über Säulenchromatographie gereinigt und in sterilem PBS in Konzentrationen von 2x10⁶ Parasiten/100 µl aliquotiert. Eine Lebendfärbung mit Trypan Blau ergab 97 % an lebensfähigen Tachyzoiten.

### Für die Prophylaxe eingesetzte Substanzen (Tabelle 2)

Toltrazuril reiner Wirkstoff wurde als Stocklösung mit 50mg/10 ml H₂O + 100 µl Cremophor angesetzt. In 0,1 ml dieser Lösung befanden sich pro Applikation 0,5 mg reiner Wirkstoff. In einer 20 g Maus entspricht dies einer Anwendungskonzentration von 25 mg/kg. Die Substanzen wurden mittels Schlundsonde peroral an 6 aufeinanderfolgenden Tagen verabreicht, dies entspricht einer Gesamtaufnahme von 150 mg/Maus.

Toltrazuril 2,5 % formulierte Lösung(100 ml Lösung enthalten: 2,50 g Toltrazuril, 30,00 g Triethanolamine, 80,70 g Polyethylene glycol; die Komponenten werden einfach gemischt.): Es wurden 6,25 ml der 2,5 %igen Lösung in 250 ml Wasser verdünnt und den Mäusen an 6 aufeinanderfolgenden Tagen über das Trinkwasser verabreicht.

### Infektion

Vier Stunden nach prophylaktischem Behandlungsbeginn wurde jede Maus mit 2x10⁶ Parasitenstadien/100 µl sterilem PBS infiziert.

### Euthanasie

14 Tage nach der Infektion wurden die Versuchsmäuse mit CO₂ getötet. Sie wurden über Randomisierung mit Nummern versehen um eine zufällige Reihenfolge bei der Auswertung einzuhalten (Blindauswertung). Die Blutentnahme erfolgte aus dem Herzmuskel für die Serumgewinnung. Das Gehirn wurde sorgfältig präpariert und eine Hälfte bei -80°C für die PCR-Analyse gespeichert. Der Rest wurde in 4 % Paraformaldehyd/PBS für Immunhistologische Untersuchungen fixiert (IFAT).

### Ergebnisse

### 1. IgG im Serum (Tabelle 3)

Anti-*N.caninum* gesamt Immunglobulin G (IgG) wurde über ELISA gemessen entsprechen der Methode von Eperon et al., 1999, Parasite Immunology 21:225-236. Behandelte Gruppen werden mit einer nichtinfizierten und einer infizierten Kontrollgruppe verglichen. Die positive Kontrollmaus wurde mit einem Rohextrakt von *N. caninum* Tachyzoiten geimpft und im folgenden mit 10⁶ *N. caninum* Stadien infiziert. In dieser Maus waren die Parasiten-spezifischen IgG Werte besonders hoch.

In Mäusen, die mit Toltrazuril (reinem Wirkstoff) behandelt wurden, zeigte sich ein verringerter Anteil von *Neospora caninum* spezifischen IgG Antikörpern als in der infizierten und unbehandelten Kontrollgruppe. Dieser Anteil war in den Mäusen, die mit Toltrazuril 2,5 % formulierter Lösung behandelt wurden, deutlich niedriger (Annäherung zur nichtinfizierten Kontrollgruppe).

### 2. PCR-Analyse (Tabelle 4)

Die Isolation von DNA aus dem Gehirn infizierter Mäuse und die Durchführung einer *Neospora caninum* spezifischen Polymerase-Kettenreaktion (PCR) wurde entsprechend Eperon et al 1999, Parasite Immunology 21:225-236, durchgeführt. Alle nichtinfizierten Mäuse waren in der *N. caninum*-specifischen PCR-Reaktion negativ. Alle infizierten Kontrollmäuse waren PCR-positiv. In der Gruppe, die mit Toltrazuril reinem Wirkstoff prophylaktisch behandelt wurde, waren 4/7 Mäusen PCR-negativ. In der Gruppe, die mit 2,5 % formulierter Toltrazuril-Lösung behandelt wurden, waren alle Mäuse PCR negativ.

### 3. Immunofluoreszenz Test (IFAT, Tabelle 4)

Paraformaldehyd fixierte Gehimproben wurden in Paraffin eingebettet und entwässert. Drei aufeinanderfolgende saggitale Schnitte wurden durchgeführt. Einer wurde mit Hematoxilin-Eosin gefärbt, die beiden anderen wurden für die Immunfluoreszenzmarkierung aufbereitet (Eperon et al., 1999. Parasite Immunology 21:225-236) mit folgenden Veränderungen: der erste Antikörper war ein polyklonaler Antikörper aus dem Kaninchen gegen ganze *N. caninum* Tachyzoiten mit einer Verdünnung von 1:400 in 1 % BSA in PBS. Der zweite Antikörper war ein Ziege-anti-Kaninchen FITC markierter Antikörper in einer Konzentration von 1:100 in 0,5 % BSA in PBS.

Die Hematoxilin-Eosin gefärbten Schnitte zeigten in allen Gruppen keinerlei Läsionen oder abnorem Veränderungen, darum wurde die Immunmarkierung mit spezifischen *N. caninum* Antikörpern hinzugezogen, um Parasitenstadien im Hirngewebe zu markieren. Bei der Beurteilung wurden die ganzen Schnitte untersucht. Auftretende Parasitenstadien wurden gezählt und folgende Bewertung angewendet:
- Bewertung (-) =: keine Tachyzoiten
- Bewertung (+) =: weniger als 10 Tachyzoiten
- Bewertung (++) =: zwischen 10 und 200 Tachyzoiten
- Bewertung (+++) =: mehr als 200 Parasiten

Die Bewertung (+++) trat nur bei der Positivkontrolle auf. Die Positivkontrolle war eine knock-out Mutante (µMT-Maus), die keine Antikörper produziert und aufgrund der Infektion am Tag 31 nach der Infektion verstarb. Alle nicht infizierten Mäuse waren im IFAT-Test negativ. Alle mit Toltrazuril behandelten Gruppen waren im IFAT-Test negativ.

**Tabelle 2:**

| Wirksamkeit von Toltrazuril gegen Neospora caninum in der Maus | | | | | |
|---|---|---|---|---|---|
| **Tag** | **Datum** | **Toltrazuril Reiner Wirkstoff 25 mg/kg/Tag p.o.** | **Toltrazuril 2,5 % Formulierung i.W.** | **Infizierte Kontrolle** | **Nichtinfizierte Kontrolle** |
| BL/6-Mäuse | | 7 Mäuse | 5 Mäuse | 5 Mäuse | 5 Mäuse |
| Tag 0 | 21.06.99 | 25 mg/Maus | 2,5 % i.W. | - | - |
| Infektion 4 h später | 21.06.99 | 2 x 10⁶ Tachyzoiten i.p. | 2 x 10⁶ Tachyzoiten i.p. | 2 x 10⁶ Tachyzoiten i.p. | Keine Infektion |
| Tag 1 p.i. | 22.06.99 | 25 mg/Maus | 2,5 % i.W | - | - |
| Tag 2 p.i. | 23.06.99 | 25 mg/Maus | 2,5 % i.W | - | - |
| Tag 3 p.i. | 24.06.99 | 25 mg/Maus | 2,5 % i.W | - | - |
| Tag 4 p.i. | 25.06.99 | 25 mg/Maus | 2,5 % i.W | - | - |
| Tag 5 p.i. | 26.06.99 | 25 mg/Maus | 2,5 % i.W | - | - |
| | | | | | |
| Tag 14 p.i. | 5.07.99 | Euthanasie | Euthanasie | Euthanasie | Euthanasie |
| p.i. = post infectionem | | | | | |
| p.o. = per oral | | | | | |
| i.W.= im Wasser | | | | | |

**Tabelle 3:**

| Anti- Neospora caninum IgG im Serum von einzelnen Mäusen, sowie Mittelwert der IgG Konzentration im Serum pro Behandlungs-Gruppe | | | |
|---|---|---|---|
| **Versuchsgruppe** | *Maus Nr*.*** | **OD (anti Neospora IgG im Serum)** | **OD Mittelwert** |
| Nicht inf. Kontrolle | 242 | 0.034 | 0.0340 (+/- 0.0044) |
| Nicht inf. Kontrolle | 228 | 0.03 | |
| Nicht inf. Kontrolle | 240 | 0.028 | |
| Nicht inf. Kontrolle | 227 | 0.045 | |
| Nicht inf. Kontrolle | 233 | 0.033 | |
| Inf. Kontrolle | 239 | 0.178 | 0.2352 (+/- 0.0542) |
| Inf. Kontrolle | 224 | 0.224 | |
| Inf. Kontrolle | 246 | 0.168 | |
| Inf. Kontrolle | 234 | 0.242 | |
| Inf. Kontrolle | 222 | 0.364 | |
| Toltrazuril 25 mg/kg | 241 | 0.338 | 0.1689 (+/-0.0807) |
| Toltrazuril 25 mg/kg | 225 | 0.271 | |
| Toltrazuril 25 mg/kg | 244 | 0.056 | |
| Toltrazuril 25 mg/kg | 220 | 0.066 | |
| Toltrazuril 25 mg/kg | 231 | 0.18 | |
| Toltrazuril 25 mg/kg | 235 | 0.103 | |
| Toltrazuril 25 mg/kg | 245 | 0.168 | |
| Toltrazuril 2,5 % i.W. | 230 | 0.085 | 0.0838 (+/- 0.0254) |
| Toltrazuril 2,5 % i.W. | 238 | 0.109 | |
| Toltrazuril 2,5 % i.W. | 243 | 0.121 | |
| Toltrazuril 2,5 % i.W. | 221 | 0.022 | |
| Toltrazuril 2,5 % i.W. | 229 | 0.082 | |
| Positivkontrolle | 14 | 0.381 | 0.381 (+/- 0) |

**Tabelle 4:**

| *N. caninum*-spezifische PCR und IFAT | | | |
|---|---|---|---|
| **Versuchsgruppe** | *Maus Nr*.* | **Neospora-PCR** | **IFAT** |
| Nicht inf. Kontrolle | 242 | - | - |
| Nicht inf. Kontrolle | 228 | - | - |
| Nicht inf. Kontrolle | 240 | - | - |
| Nicht inf. Kontrolle | 227 | - | - |
| Nicht inf. Kontrolle | 233 | - | - |
| Inf. Kontrolle | 239 | + | + |
| Inf. Kontrolle | 224 | + | + |
| Inf. Kontrolle | 246 | + | ++ |
| Inf. Kontrolle | 234 | + | - |
| Inf. Kontrolle | 222 | + | + |
| Toltrazuril 25 mg/kg | 241 | + | - |
| Tolrrazuril 25 mg/kg | 225 | - | - |
| Toltrazuril 25 mg/kg | 244 | - | - |
| Toltrazuril 25 mg/kg | 220 | - | - |
| Toltrazuril 25 mg/kg | 231 | + | - |
| Toltrazuril 25 mg/kg | 235 | - | - |
| Toltrazuril 25 mg/kg | 245 | + | - |
| Toltrazuril 2,5 % i.W. | 230 | - | - |
| Toltrazuril 2,5 % i.W. | 238 | - | - |
| Toltrazuril 2,5 % i.W. | 243 | - | - |
| Toltrazuril 2,5 % i.W. | 221 | - | - |
| Toltrazuril 2,5 % i.W. | 229 | - | - |
| Positivkontrolle | 14 | + | +++ |

| | | | |
|---|---|---|---|
| * Die Nummern wurden durch Randomisieren vergeben, um eine unbeeinflusste Auswertung zu gewährleisten, daher erscheint hier keine logische Abfolge. | | | |

## Patentansprüche

1. Verwendung von Toltrazuril oder Ponazuril zur Herstellung von Mitteln zur prophylaktischen Behandlung von Tieren gegen *Neospora caninum*.

2. Verwendung von Toltrazuril oder Ponazuril zur Herstellung von Mitteln zur Anwendung in Kombination mit Lebend- oder Totvakzinen bei der Behandlung von Tieren gegen parasitäre Protozoen.

3. Mittel enthaltend einen Triazinon-Wirkstoff ausgewählt aus Ponazuril und Toltrazuril sowie eine Lebend- oder Totvaccine als Kombinationspräparat gegen parasitäre Protozoen.

## Claims

1. Use of toltrazuril or ponazuril for the preparation of compositions for the prophylactic treatment of animals against *Neospora caninum*.

2. Use of toltrazuril or ponazuril for the preparation of compositions for use in combination with live or dead vaccines for treating animals against parasitic protozoans.

3. Compositions comprising a triazinone active compound selected from ponazuril and toltrazuril and a live or dead vaccine as combination preparation against parasitic protozoans.

## Revendications

1. Utilisation de toltrazuril ou de ponazuril pour la préparation d'agents destinés à traiter de façon prophylactique des animaux contre le *Neospora caninum*.

2. Utilisation de toltrazuril ou de ponazuril pour la préparation d'agents pour l'application en combinaison avec des vaccins vivants ou inactivés pour traiter des animaux contre des protozoaires parasitaires.

3. Agent contenant un principe actif de triazinone choisi parmi le ponazuril et le toltrazuril ainsi qu'un vaccin vivant ou inactivé sous forme de préparation combinée contre des protozoaires parasitaires.
